# EUROPEAN PATENT APPLICATION

(11) **EP 4 681 707 A1**
(43) Date of publication of application: **21.01.2026**
(21) Application number: 24770023.0
(22) Date of filing: 15.03.2024
(51) Int. Cl.: A61K 31/165, A61K 39/395, A61P 35/00

(54) **METHOD FOR TREATING DISEASES BY USING REPARIXIN ALONE OR IN COMBINATION WITH ANTI-PD-1 ANTIBODY**

(30) Priority: 15.03.2023 CN 202310249973
(71) Applicant: Shandong Cancer Hospital Affiliated to Shandong First Medical University, Jinan, Shandong 250117 (CN)
(72) Inventor: ZHAO, Lei, Jinan, Shandong 250001 (CN); YANG, Yue, Jinan, Shandong 250001 (CN); CHEN, Yunsong, Jinan, Shandong 250001 (CN)
(74) Representative: Gille Hrabal Partnerschaftsgesellschaft mbB Patentanwälte
(86) International application number: PCT/CN2024/081852
(87) International publication number: WO 2024/188333

(57) **Abstract**

Provided in the present invention is a new pharmaceutical use for Reparixin, said use comprising use in the preparation of a drug for treating and/or preventing colorectal cancer liver metastases (CRLM) (especially CRLM associated with NAFLD), and use in the preparation of a drug for treating and/or preventing non-alcoholic fatty liver disease (NAFLD). Further provided in the present invention is a pharmaceutical combination composition comprising an anti-PD-1 antibody and Reparixin. The pharmaceutical combination composition can be used for treating and/or preventing cancers, especially CRLM associated with NAFLD and lung cancer liver metastases associated with NAFLD.

## Description

The present application claims priority to prior application No. 202310249973.8 filed with China National Intellectual Property Administration on Mar. 15, 2023 and entitled "METHOD FOR TREATING DISEASES BY USING REPARIXIN ALONE OR IN COMBINATION WITH ANTI-PD-1 ANTIBODY", which is incorporated herein by reference in its entirety.

### TECHNICAL FIELD

The present disclosure relates to the technical field of pharmaceuticals, and in particular to a method of treatment of a disease with reparixin alone or in combination with an anti-PD-1 antibody.

### BACKGROUND

At present, the number of NAFLD patients in China ranks first in the world, with a prevalence rate as high as 29.2%[1]. Within only ten years, the prevalence rate of NAFLD in China has risen from 18% to 29%, with a growth rate that is no less than twice that of Western countries[2, 3]. By 2030, China will remain the country with the fastest-growing prevalence rate among countries with a high incidence of NAFLD, with the number of cases projected to increase by 29.1%[4].

Based on an estimated prevalence rate of 29%, there were 151,232 new CRC cases complicated by NAFLD in China in 2018, which was very close to the total number of new CRC cases in the United States, which ranked second. The actual number of cases will exceed this figure, as the prevalence rate of NAFLD is higher in the 50-69 age group with a high incidence of CRC[1].

At present, the first-line treatment of NASH and NAFLD mainly includes lifestyle intervention therapies, such as dietary calorie restriction and exercise. For obese patients who have not yet achieved disease remission after 6 months of lifestyle intervention, bariatric surgery may be considered.

The development of therapeutic drugs for NAFLD has been vigorously developed in the past 10 years, mainly aiming at metabolic syndrome and liver injury. Common drugs include antioxidants, metabolic modifiers, anti-fibrosis drugs, and the like, but there is no approved drug treatment regimen at present. Drugs for correcting abnormal lipid metabolism include acetyl-CoA carboxylase (ACC) inhibitors, stearoyl-CoA desaturase-1 (SCD1) inhibitors, fatty acid synthase (FASN) inhibitors, peroxisome proliferator-activated receptor (PPAR)-α/δ agonists, thyroid hormone receptor β (THRβ) agonists, and the like.

The liver is the organ most commonly affected by malignant tumor metastasis among parenchymal organs (6); colorectal cancer (CRC) is the most common primary tumor source of metastatic liver cancer. Retrospective analysis of a large number of clinical cases has confirmed that when the primary tumor of CRC is detected, 8%-9% of patients are diagnosed with synchronous colorectal liver metastases (synCRLM)[5, 6](7,8). Additionally, 20%-33% of patients develop metachronous liver metastases (metCRLM) during subsequent treatment[7, 8](9,10). Ultimately, more than half of CRC patients die directly from liver metastasis. Therefore, CRLM is a key factor affecting the therapeutic effect and prognosis of CRC.

In terms of clinical studies, a retrospective analysis of CRC patients who underwent surgery between 1990 and 2014 showed that there was no significant difference in the incidence of liver metastasis between the NASH group and the non-NASH group[9]. Results from another prospective clinical study indicated that after radical resection of CRLM, fatty liver disease (FLD; the authors did not distinguish it from NAFLD) was an independent risk factor for local liver recurrence[10].

In general, it is relatively clear that concurrent NAFLD can promote the development and progression of CRC. In recent years, attention has been paid to the specific impact of NAFLD on CRLM, but there is no universally accepted conclusion, requiring further in-depth study[11]. If these specific patients can be accurately identified and treated accordingly, they will benefit.

Immune checkpoint inhibition (ICI) in combination with programmed cell death protein 1 (PD-1) retardants is effective in patients with microsatellite instability-high (MSI-H) CRC, but the proportion is less than 5% of patients with locally advanced or metastatic CRC[12]. The objective response rate of ICI was less than 5% in all CRC patients[13, 14]. However, tumor-infiltrating lymphocytes are associated with the prognosis. Immune scoring (grading the density of CD3⁺ and CD8⁺ T cells in CRLM patients) is associated with a relatively good prognosis[15], and high regulatory T cell (Treg cell) infiltration is associated with a relatively short OS[16]. Both the density and morphology of tumor-associated macrophages (TAMs) are associated with the survival of CRLM patients[17, 18]. Thus, there is a need for effective immunotherapy to enhance the impaired tumor immune microenvironment in CRLM. Similarly, in *in vivo* experiments in mice, PD-1 monotherapy has no inhibitory effect on liver tumor growth. The relatively poor response of liver cancer to PD-1 treatment is mainly attributed to the unique hepatic immune tolerance microenvironment, independent of tumor origin or type[19].

Reparixin has been shown to reduce the inflammatory response in various injury models. Spontaneously hypertensive rats (SHRs) were subcutaneously injected with reparixin (5 mg/kg) once daily for 3 consecutive weeks, which effectively reduced the systolic blood pressure and increased the blood flow[20]. Reparixin can reduce the level of IL-1β in the brain after middle cerebral artery occlusion/reperfusion (MCAo) in mice[21]. In addition, recent basic studies have shown that reparixin prolongs overall survival (OS) rate in the treatment of gastric cancer, drives the reduction of programmed death ligand 1 macrophages, and promotes anti-tumor immunity[22].

Hepatocellular carcinoma is a malignant tumor with a high incidence and high invasiveness and metastatic potential in China. Li et al. found that blocking the CCL2-CCR2 signal can inhibit the infiltration and M2 polarization of TAMs in tumor tissues and activate CD8+ T cells. By establishing various liver cancer models of mouse liver cancer cells Hepa1-6, LPC-H12, and H22 (a homologous mouse orthotopic liver cancer model, a postsurgical recurrence model, and a subcutaneous inoculation model), treatment with the CCR2 antagonist RDC018 (30 mg/kg) or blocking the CCL2-CCR2 signal using CCR2 gene knockout mice significantly inhibited tumor growth and metastasis, reduced postoperative recurrence, and prolonged survival[23]. Professor Loberg et al. reported that in SCID mice (with T-cell and B-cell immunodeficiency) subcutaneously injected with prostate cancer cell VCap, the human CCL2-targeting neutralizing antibody CNTO888 or mouse CCL2-targeting neutralizing antibody C1142 can significantly inhibit the growth of subcutaneous graft tumors, and reduce the infiltration of CD68⁺ macrophages and the formation of tumor microvessels[24]. Dual treatment with CCR2a and anti-pd-1 can greatly inhibit the growth of residual tumors and reduce distant metastasis; compared with CCR2a and anti-PD-1 monotherapy, the survival of mice receiving dual treatment is significantly prolonged, and the number and cytolytic function of CD8+ cells in dual-treated mice are significantly increased. Preclinical animal experimental studies have shown that targeting the CCL2-CCR2 axis has significant anti-tumor growth and anti-metastasis effects. A preclinical study evaluating the effects of CCR2 antagonist RDC018 on hepatocellular carcinoma showed that RDC018 inhibited tumor growth and metastasis, reduced postsurgical recurrence, and prolonged survival. At present, many small molecule immunotherapies have entered the clinical development stage and can be used as monotherapy or in combination with antibody drugs and conventional chemotherapeutic drugs to further improve anti-tumor efficacy or ameliorate the problem of resistance to checkpoint inhibitors.

### SUMMARY

In order to solve the technical problems described above, in a first aspect, the present disclosure provides use of reparixin or a pharmaceutically acceptable salt thereof for the manufacturing of a medicament for the treatment and/or prevention of colorectal cancer liver metastasis CRLM and liver metastasis from lung cancer LCLM.

In some embodiments, the colorectal cancer liver metastasis is colorectal cancer liver metastasis complicated by NAFLD.

In some embodiments, the liver metastasis from lung cancer is liver metastasis from lung cancer complicated by NAFLD.

In some embodiments, the pharmaceutically acceptable salt of reparixin is selected from a lysine salt and a sodium salt.

In some embodiments, reparixin or the pharmaceutically acceptable salt thereof is administered to a human subject at a dose of 0.1-10.0 mg/kg according to the body weight of the patient; the dose may be 0.1 mg/kg, 0.2 mg/kg, 0.3 mg/kg, 0.4 mg/kg, 0.5 mg/kg, 0.6 mg/kg, 0.7 mg/kg, 0.8 mg/kg, 0.9 mg/kg, 1.0 mg/kg, 1.2 mg/kg, 1.4 mg/kg, 1.6 mg/kg, 1.8 mg/kg, 2.0 mg/kg, 2.2 mg/kg, 2.4 mg/kg, 2.6 mg/kg, 2.8 mg/kg, 3.0 mg/kg, 3.2 mg/kg, 3.4 mg/kg, 3.6 mg/kg, 3.8 mg/kg, 4.0 mg/kg, 4.2 mg/kg, 4.4 mg/kg, 4.6 mg/kg, 4.8 mg/kg, 5.0 mg/kg, 5.2 mg/kg, 5.4 mg/kg, 5.6 mg/kg, 5.8 mg/kg, 6.0 mg/kg, 6.2 mg/kg, 6.4 mg/kg, 6.6 mg/kg, 6.8 mg/kg, 7.0 mg/kg, 7.2 mg/kg, 7.4 mg/kg, 7.6 mg/kg, 7.8 mg/kg, 8.0 mg/kg, 8.2 mg/kg, 8.4 mg/kg, 8.6 mg/kg, 8.8 mg/kg, 9.0 mg/kg, 9.2 mg/kg, 9.4 mg/kg, 9.6 mg/kg, 9.8 mg/kg, 10.0 mg/kg, or any value between any two of these values.

In an alternative embodiment, reparixin or the pharmaceutically acceptable salt thereof is administered to a human subject at a dose of 0.1-100 mg; the dose may be 0.1 mg, 0.2 mg, 0.3 mg, 0.4 mg, 0.5 mg, 0.6 mg, 0.7 mg, 0.8 mg, 0.9 mg, 1.0 mg, 2 mg, 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, 30 mg, 31 mg, 32 mg, 33 mg, 34 mg, 35 mg, 36 mg, 37 mg, 38 mg, 39 mg, 40 mg, or any value between any two of these values.

In a second aspect, the present disclosure further provides a method of treatment and/or prevention of colorectal cancer liver metastasis CRLM and liver metastasis from lung cancer LCLM, which comprises administering to a patient reparixin or a pharmaceutically acceptable salt thereof.

In some embodiments, the colorectal cancer liver metastasis is colorectal cancer liver metastasis complicated by NAFLD.

In some embodiments, the liver metastasis from lung cancer is liver metastasis from lung cancer complicated by NAFLD.

In some embodiments, the pharmaceutically acceptable salt of reparixin is selected from a lysine salt and a sodium salt.

In some embodiments, reparixin or the pharmaceutically acceptable salt thereof is administered to a human subject at a dose of 0.1-10.0 mg/kg according to the body weight of the patient; the dose may be 0.1 mg/kg, 0.2 mg/kg, 0.3 mg/kg, 0.4 mg/kg, 0.5 mg/kg, 0.6 mg/kg, 0.7 mg/kg, 0.8 mg/kg, 0.9 mg/kg, 1.0 mg/kg, 1.2 mg/kg, 1.4 mg/kg, 1.6 mg/kg, 1.8 mg/kg, 2.0 mg/kg, 2.2 mg/kg, 2.4 mg/kg, 2.6 mg/kg, 2.8 mg/kg, 3.0 mg/kg, 3.2 mg/kg, 3.4 mg/kg, 3.6 mg/kg, 3.8 mg/kg, 4.0 mg/kg, 4.2 mg/kg, 4.4 mg/kg, 4.6 mg/kg, 4.8 mg/kg, 5.0 mg/kg, 5.2 mg/kg, 5.4 mg/kg, 5.6 mg/kg, 5.8 mg/kg, 6.0 mg/kg, 6.2 mg/kg, 6.4 mg/kg, 6.6 mg/kg, 6.8 mg/kg, 7.0 mg/kg, 7.2 mg/kg, 7.4 mg/kg, 7.6 mg/kg, 7.8 mg/kg, 8.0 mg/kg, 8.2 mg/kg, 8.4 mg/kg, 8.6 mg/kg, 8.8 mg/kg, 9.0 mg/kg, 9.2 mg/kg, 9.4 mg/kg, 9.6 mg/kg, 9.8 mg/kg, 10.0 mg/kg, or any value between any two of these values.

In an alternative embodiment, reparixin or the pharmaceutically acceptable salt thereof is administered to a human subject at a dose of 0.1-100 mg; the dose may be 0.1 mg, 0.2 mg, 0.3 mg, 0.4 mg, 0.5 mg, 0.6 mg, 0.7 mg, 0.8 mg, 0.9 mg, 1.0 mg, 2 mg, 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, 30 mg, 31 mg, 32 mg, 33 mg, 34 mg, 35 mg, 36 mg, 37 mg, 38 mg, 39 mg, 40 mg, or any value between any two of these values.

In a third aspect, the present disclosure provides use of reparixin or a pharmaceutically acceptable salt thereof for the manufacturing of a medicament for the treatment and/or prevention of non-alcoholic fatty liver disease NAFLD.

In some embodiments, the non-alcoholic fatty liver disease includes primary non-alcoholic fatty liver disease and secondary non-alcoholic fatty liver disease.

In some embodiments, the non-alcoholic fatty liver disease includes simple fatty liver, non-alcoholic steatohepatitis, and non-alcoholic steatohepatitis-related cirrhosis.

In some embodiments, the pharmaceutically acceptable salt of reparixin is selected from a lysine salt and a sodium salt.

In some embodiments, reparixin or the pharmaceutically acceptable salt thereof is administered to a human subject at a dose of 0.1-10.0 mg/kg according to the body weight of the patient; the dose may be 0.1 mg/kg, 0.2 mg/kg, 0.3 mg/kg, 0.4 mg/kg, 0.5 mg/kg, 0.6 mg/kg, 0.7 mg/kg, 0.8 mg/kg, 0.9 mg/kg, 1.0 mg/kg, 1.2 mg/kg, 1.4 mg/kg, 1.6 mg/kg, 1.8 mg/kg, 2.0 mg/kg, 2.2 mg/kg, 2.4 mg/kg, 2.6 mg/kg, 2.8 mg/kg, 3.0 mg/kg, 3.2 mg/kg, 3.4 mg/kg, 3.6 mg/kg, 3.8 mg/kg, 4.0 mg/kg, 4.2 mg/kg, 4.4 mg/kg, 4.6 mg/kg, 4.8 mg/kg, 5.0 mg/kg, 5.2 mg/kg, 5.4 mg/kg, 5.6 mg/kg, 5.8 mg/kg, 6.0 mg/kg, 6.2 mg/kg, 6.4 mg/kg, 6.6 mg/kg, 6.8 mg/kg, 7.0 mg/kg, 7.2 mg/kg, 7.4 mg/kg, 7.6 mg/kg, 7.8 mg/kg, 8.0 mg/kg, 8.2 mg/kg, 8.4 mg/kg, 8.6 mg/kg, 8.8 mg/kg, 9.0 mg/kg, 9.2 mg/kg, 9.4 mg/kg, 9.6 mg/kg, 9.8 mg/kg, 10.0 mg/kg, or any value between any two of these values.

In an alternative embodiment, reparixin or the pharmaceutically acceptable salt thereof is administered to a human subject at a dose of 0.1-100 mg; the dose may be 0.1 mg, 0.2 mg, 0.3 mg, 0.4 mg, 0.5 mg, 0.6 mg, 0.7 mg, 0.8 mg, 0.9 mg, 1.0 mg, 2 mg, 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, 30 mg, 31 mg, 32 mg, 33 mg, 34 mg, 35 mg, 36 mg, 37 mg, 38 mg, 39 mg, 40 mg, or any value between any two of these values.

In a fourth aspect, the present disclosure further provides a method of treatment and/or prevention of non-alcoholic fatty liver disease NAFLD, which comprises administering to a patient reparixin or a pharmaceutically acceptable salt thereof.

In some embodiments, the non-alcoholic fatty liver disease includes primary non-alcoholic fatty liver disease and secondary non-alcoholic fatty liver disease.

In some embodiments, the non-alcoholic fatty liver disease includes simple fatty liver, non-alcoholic steatohepatitis, and non-alcoholic steatohepatitis-related cirrhosis.

In some embodiments, the pharmaceutically acceptable salt of reparixin is selected from a lysine salt and a sodium salt.

In some embodiments, reparixin or the pharmaceutically acceptable salt thereof is administered to a human subject at a dose of 0.1-10.0 mg/kg according to the body weight of the patient; the dose may be 0.1 mg/kg, 0.2 mg/kg, 0.3 mg/kg, 0.4 mg/kg, 0.5 mg/kg, 0.6 mg/kg, 0.7 mg/kg, 0.8 mg/kg, 0.9 mg/kg, 1.0 mg/kg, 1.2 mg/kg, 1.4 mg/kg, 1.6 mg/kg, 1.8 mg/kg, 2.0 mg/kg, 2.2 mg/kg, 2.4 mg/kg, 2.6 mg/kg, 2.8 mg/kg, 3.0 mg/kg, 3.2 mg/kg, 3.4 mg/kg, 3.6 mg/kg, 3.8 mg/kg, 4.0 mg/kg, 4.2 mg/kg, 4.4 mg/kg, 4.6 mg/kg, 4.8 mg/kg, 5.0 mg/kg, 5.2 mg/kg, 5.4 mg/kg, 5.6 mg/kg, 5.8 mg/kg, 6.0 mg/kg, 6.2 mg/kg, 6.4 mg/kg, 6.6 mg/kg, 6.8 mg/kg, 7.0 mg/kg, 7.2 mg/kg, 7.4 mg/kg, 7.6 mg/kg, 7.8 mg/kg, 8.0 mg/kg, 8.2 mg/kg, 8.4 mg/kg, 8.6 mg/kg, 8.8 mg/kg, 9.0 mg/kg, 9.2 mg/kg, 9.4 mg/kg, 9.6 mg/kg, 9.8 mg/kg, 10.0 mg/kg, or any value between any two of these values.

In an alternative embodiment, reparixin or the pharmaceutically acceptable salt thereof is administered to a human subject at a dose of 0.1-100 mg; the dose may be 0.1 mg, 0.2 mg, 0.3 mg, 0.4 mg, 0.5 mg, 0.6 mg, 0.7 mg, 0.8 mg, 0.9 mg, 1.0 mg, 2 mg, 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, 30 mg, 31 mg, 32 mg, 33 mg, 34 mg, 35 mg, 36 mg, 37 mg, 38 mg, 39 mg, 40 mg, or any value between any two of these values.

In a fifth aspect, the present disclosure further provides a pharmaceutical combination composition comprising an anti-PD-1 antibody or an antigen-binding fragment thereof and reparixin or a pharmaceutically acceptable salt thereof.

In some embodiments, the anti-PD-1 antibody or the antigen-binding fragment thereof has a synergistic pharmacodynamic effect with reparixin or the pharmaceutically acceptable salt thereof.

In some embodiments, the pharmaceutically acceptable salt of reparixin is selected from a lysine salt and a sodium salt.

In some embodiments, the PD-1 antibody is a PD-1 monoclonal antibody.

In some embodiments, a light chain variable region of the PD-1 antibody comprises an LCDR1, an LCDR2, and an LCDR3 set forth in SEQ ID NO: 4, SEQ ID NO: 5, and SEQ ID NO: 6, respectively. In some embodiments, a heavy chain variable region of the PD-1 antibody comprises an HCDR1, an HCDR2, and an HCDR3 set forth in SEQ ID NO: 1, SEQ ID NO: 2, and SEQ ID NO: 3, respectively. The CDR sequences described above are shown in the table below:

| Name | Sequence | No. |
|---|---|---|
| HCDR1 | SYMMS | SEQ ID NO:1 |
| HCDR2 | TISGGGANTYYPDSVKG | SEQ ID NO:2 |
| HCDR3 | QLYYFDY | SEQ ID NO:3 |
| LCDR1 | LASQTIGTWLT | SEQ ID NO:4 |
| LCDR2 | TATSLAD | SEQ ID NO:5 |
| LCDR3 | QQVYSIPWT | SEQ ID NO:6 |

In some embodiments, the PD-1 antibody is a humanized antibody.

In some embodiments, the humanized antibody comprises the light chain variable region set forth in SEQ ID NO: 10 or a variant thereof, wherein the variant preferably has 0-10 amino acid changes, more preferably an amino acid change of A43S, in the light chain variable region set forth in SEQ ID NO: 10; the humanized antibody comprises the heavy chain variable region set forth in SEQ ID NO: 9 or a variant thereof, wherein the variant preferably has 0-10 amino acid changes, more preferably an amino acid change of G44R, in the heavy chain variable region set forth in SEQ ID NO: 9.

The sequences of the heavy and light chain variable regions of the aforementioned humanized antibody are shown below:
Heavy chain variable region, SEQ ID NO: 9, 
EVQLVESGGGLVQPGGSLRLSCAASGFTFSSYMMSWVRQAPGKGLEWVATISG
GGANTYYPDSVKGRFTISRDNAKNSLYLQMNSLRAEDTAVYYCARQLYYFDY
WGQGTTVTVSS
;
Light chain variable region, SEQ ID NO: 10,

DIQMTQSPSSLSASVGDRVTITCLASQTIGTWLTWYQQKPGKAPKLLIYTATSLA
DGVPSRFSGSGSGTDFTLTISSLQPEDFATYYCQQVYSIPWTFGGGTKVEIK

In some embodiments, the humanized antibody comprises the light chain set forth in SEQ ID NO: 8 or a variant thereof, wherein the variant preferably has 0-10 amino acid changes, more preferably an amino acid change of A43S, in the light chain variable region; the humanized antibody comprises the heavy chain set forth in SEQ ID NO: 7 or a variant thereof, wherein the variant preferably has 0-10 amino acid changes, more preferably an amino acid change of G44R, in the heavy chain variable region.

In some embodiments, the humanized antibody comprises the light chain set forth in SEQ ID NO: 8 and the heavy chain set forth in SEQ ID NO: 7.

The sequences of the heavy and light chains of the humanized antibody are shown below:
Heavy chain, SEQ ID NO: 7, 
EVQLVESGGGLVQPGGSLRLSCAASGFTFSSYMMSWVRQAPGKGLEWVATISG
GGANTYYPDSVKGRFTISRDNAKNSLYLQMNSLRAEDTAVYYCARQLYYFDY
WGQGTTVTVSSASTKGPSVFPLAPCSRSTSESTAALGCLVKDYFPEPVTVSWNS
GALTSGVHTFPAVLQSSGLYSLSSVVTVPSSSLGTKTYTCNVDHKPSNTKVDKR
VESKYGPPCPPCPAPEFLGGPSVFLFPPKPKDTLMISRTPEVTCVVVDVSQEDPE
VQFNWYVDGVEVHNAKTKPREEQFNSTYRVVSVLTVLHQDWLNGKEYKCKV
SNKGLPSSIEKTISKAKGQPREPQVYTLPPSQEEMTKNQVSLTCLVKGFYPSDIAV
EWESNGQPENNYKTTPPVLDSDGSFFLYSRLTVDKSRWQEGNVFSCSVMHEAL
HNHYTQKSLSLSLGK ;
Light chain, SEQ ID NO: 8,

DIQMTQSPSSLSASVGDRVTITCLASQTIGTWLTWYQQKPGKAPKLLIYTATSLA
DGVPSRFSGSGSGTDFTLTISSLQPEDFATYYCQQVYSIPWTFGGGTKVEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC

In some embodiments, the anti-PD-1 antibody or the antigen-binding fragment thereof is administered to a human subject at a dose of 0.1-10.0 mg/kg according to the body weight of the patient; the dose may be 0.1 mg/kg, 0.2 mg/kg, 0.3 mg/kg, 0.4 mg/kg, 0.5 mg/kg, 0.6 mg/kg, 0.7 mg/kg, 0.8 mg/kg, 0.9 mg/kg, 1.0 mg/kg, 1.2 mg/kg, 1.4 mg/kg, 1.6 mg/kg, 1.8 mg/kg, 2.0 mg/kg, 2.2 mg/kg, 2.4 mg/kg, 2.6 mg/kg, 2.8 mg/kg, 3.0 mg/kg, 3.2 mg/kg, 3.4 mg/kg, 3.6 mg/kg, 3.8 mg/kg, 4.0 mg/kg, 4.2 mg/kg, 4.4 mg/kg, 4.6 mg/kg, 4.8 mg/kg, 5.0 mg/kg, 5.2 mg/kg, 5.4 mg/kg, 5.6 mg/kg, 5.8 mg/kg, 6.0 mg/kg, 6.2 mg/kg, 6.4 mg/kg, 6.6 mg/kg, 6.8 mg/kg, 7.0 mg/kg, 7.2 mg/kg, 7.4 mg/kg, 7.6 mg/kg, 7.8 mg/kg, 8.0 mg/kg, 8.2 mg/kg, 8.4 mg/kg, 8.6 mg/kg, 8.8 mg/kg, 9.0 mg/kg, 9.2 mg/kg, 9.4 mg/kg, 9.6 mg/kg, 9.8 mg/kg, 10.0 mg/kg, or any value between any two of these values.

In an alternative embodiment, the anti-PD-1 antibody or the antigen-binding fragment thereof is administered to a human subject at a dose of 10-300 mg; the dose may be 10.0 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 105 mg, 110 mg, 115 mg, 120 mg, 125 mg, 130 mg, 135 mg, 140 mg, 145 mg, 150 mg, 155 mg, 160 mg, 165 mg, 170 mg, 175 mg, 180 mg, 185 mg, 190 mg, 195 mg, 200 mg, 205 mg, 210 mg, 215 mg, 220 mg, 225 mg, 230 mg, 235 mg, 240 mg, 245 mg, 250 mg, 255 mg, 260 mg, 265 mg, 270 mg, 275 mg, 280 mg, 285 mg, 290 mg, 295 mg, 300 mg, or any value between any two of these values, preferably 50-300 mg, and most preferably 200 mg.

In some embodiments, reparixin or the pharmaceutically acceptable salt thereof is administered to a human subject at a dose of 0.1-10.0 mg/kg according to the body weight of the patient; the dose may be 0.1 mg/kg, 0.2 mg/kg, 0.3 mg/kg, 0.4 mg/kg, 0.5 mg/kg, 0.6 mg/kg, 0.7 mg/kg, 0.8 mg/kg, 0.9 mg/kg, 1.0 mg/kg, 1.2 mg/kg, 1.4 mg/kg, 1.6 mg/kg, 1.8 mg/kg, 2.0 mg/kg, 2.2 mg/kg, 2.4 mg/kg, 2.6 mg/kg, 2.8 mg/kg, 3.0 mg/kg, 3.2 mg/kg, 3.4 mg/kg, 3.6 mg/kg, 3.8 mg/kg, 4.0 mg/kg, 4.2 mg/kg, 4.4 mg/kg, 4.6 mg/kg, 4.8 mg/kg, 5.0 mg/kg, 5.2 mg/kg, 5.4 mg/kg, 5.6 mg/kg, 5.8 mg/kg, 6.0 mg/kg, 6.2 mg/kg, 6.4 mg/kg, 6.6 mg/kg, 6.8 mg/kg, 7.0 mg/kg, 7.2 mg/kg, 7.4 mg/kg, 7.6 mg/kg, 7.8 mg/kg, 8.0 mg/kg, 8.2 mg/kg, 8.4 mg/kg, 8.6 mg/kg, 8.8 mg/kg, 9.0 mg/kg, 9.2 mg/kg, 9.4 mg/kg, 9.6 mg/kg, 9.8 mg/kg, 10.0 mg/kg, or any value between any two of these values.

In an alternative embodiment, reparixin or the pharmaceutically acceptable salt thereof is administered to a human subject at a dose of 0.1-100 mg; the dose may be 0.1 mg, 0.2 mg, 0.3 mg, 0.4 mg, 0.5 mg, 0.6 mg, 0.7 mg, 0.8 mg, 0.9 mg, 1.0 mg, 2 mg, 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, 30 mg, 31 mg, 32 mg, 33 mg, 34 mg, 35 mg, 36 mg, 37 mg, 38 mg, 39 mg, 40 mg, or any value between any two of these values, preferably 1-20 mg.

In a preferred embodiment of the present disclosure, the anti-PD-1 antibody is administered by injection, e.g., subcutaneous or intravenous injection, and prior to the injection, the anti-PD-1 antibody is formulated into an injectable form. A particularly preferred injectable form of the anti-PD-1 antibody is an injectable liquid or a lyophilized powder for injection comprising the anti-PD-1 antibody, a buffering agent, and a stabilizer, and optionally further comprising a surfactant. The buffering agent may be selected from one or more of acetate, citrate, succinate, and phosphate. The stabilizer may be selected from sugars and amino acids, preferably disaccharides, such as sucrose, lactose, trehalose, and maltose. The surfactant is selected from polyoxyethylene hydrogenated castor oil, glycerol fatty acid esters, and polyoxyethylene sorbitan fatty acid esters; preferably, the polyoxyethylene sorbitan fatty acid ester is polysorbate 20, polysorbate 40, polysorbate 60, or polysorbate 80; most preferably, the polyoxyethylene sorbitan fatty acid ester is polysorbate 20. The most preferred injectable form of the anti-PD-1 antibody comprises the anti-PD-1 antibody, an acetate buffering agent, trehalose, and polysorbate 20.

In some embodiments, the pharmaceutical combination composition is packaged in a kit, and the kit further comprises an instruction for use of the anti-PD-1 antibody in combination with reparixin or the pharmaceutically acceptable salt thereof for the treatment of a cancer in a patient.

In some embodiments, in the pharmaceutical combination composition, the anti-PD-1 antibody and reparixin or the pharmaceutically acceptable salt thereof are packaged separately in their respective kits, and the kit further comprises an instruction for use of the anti-PD-L1 antibody in combination with reparixin or the pharmaceutically acceptable salt thereof for the treatment of a cancer in a patient. In some embodiments, the pharmaceutical combination composition comprises a pharmaceutical composition of the anti-PD-1 antibody and a pharmaceutical composition of reparixin or the pharmaceutically acceptable salt thereof.

In some embodiments, in the pharmaceutical combination composition, the anti-PD-1 antibody and reparixin or the pharmaceutically acceptable salt thereof are each in the form of a pharmaceutical composition and can be administered simultaneously, sequentially, or at intervals.

In some embodiments, the pharmaceutical combination composition is used for the treatment and/or prevention of a cancer.

In some embodiments, the cancer includes liver cancer, colorectal cancer, colon cancer, and rectal cancer.

In some embodiments, the cancer includes metastatic liver cancer and secondary liver cancer.

In some embodiments, the metastatic liver cancer includes colorectal cancer liver metastasis CRLM, breast cancer liver metastasis, ovarian cancer liver metastasis, neuroendocrine cancer liver metastasis, and liver metastasis from lung cancer.

In some embodiments, the cancer is colorectal cancer liver metastasis CRLM complicated by NAFLD.

In some embodiments, the pharmaceutical combination composition increases a CD8+ content in the liver.

In a sixth aspect, the present disclosure further provides use of the pharmaceutical combination composition described above for the manufacturing of a medicament for the treatment and/or prevention of a cancer.

In some embodiments, the cancer is liver cancer, colorectal cancer, colon cancer, or rectal cancer.

In some embodiments, the cancer is metastatic liver cancer or secondary liver cancer.

In some embodiments, the metastatic liver cancer includes colorectal cancer liver metastasis CRLM, breast cancer liver metastasis, ovarian cancer liver metastasis, neuroendocrine cancer liver metastasis, and liver metastasis from lung cancer LCLM.

In some embodiments, the cancer is colorectal cancer liver metastasis CRLM complicated by NAFLD. In some embodiments, the liver metastasis from lung cancer is liver metastasis from lung cancer complicated by NAFLD.

In a seventh aspect, the present disclosure further provides a method of treatment of a cancer, which comprises administering to a patient the pharmaceutical combination composition described above.

In some embodiments, the cancer is liver cancer, colorectal cancer, colon cancer, or rectal cancer.

In some embodiments, the cancer is metastatic liver cancer or secondary liver cancer.

In some embodiments, the metastatic liver cancer includes colorectal cancer liver metastasis CRLM, breast cancer liver metastasis, ovarian cancer liver metastasis, neuroendocrine cancer liver metastasis, and liver metastasis from lung cancer LCLM.

In some embodiments, the cancer is colorectal cancer liver metastasis CRLM complicated by NAFLD. In some embodiments, the liver metastasis from lung cancer is liver metastasis from lung cancer complicated by NAFLD.

### Beneficial Effects

The present disclosure provides new pharmaceutical use of reparixin, which comprises use for the manufacturing of a medicament for the treatment and/or prevention of colorectal cancer liver metastasis CRLM and liver metastasis from lung cancer LCLM (particularly colorectal cancer liver metastasis complicated by NAFLD and liver metastasis from lung cancer complicated by NAFLD), and use for the manufacturing of a medicament for the treatment and/or prevention of non-alcoholic fatty liver disease NAFLD.

The present disclosure further provides a pharmaceutical combination composition comprising an anti-PD-1 antibody and reparixin. The pharmaceutical combination composition can be used for treating and/or preventing a cancer, particularly colorectal cancer liver metastasis (CRLM) complicated by NAFLD and liver metastasis from lung cancer complicated by NAFLD.

### BRIEF DESCRIPTION OF THE DRAWINGS

FIG. 1 is a flowchart of modeling of the mouse NAFLD + CRC liver metastasis dual model.
FIG. 2 shows the body weight of NAFLD modeling mice.
FIG. 3 shows the gross anatomical views of livers (upper panel) and HE staining (lower panel) of each group of mouse models.
FIG. 4 shows a comparative analysis of liver metastases of mice across the groups.
FIG. 5 shows the screening and identification of chemokines.
FIG. 6 shows the expression of CXCL5 in the livers of each group of mice.
FIG. 7 shows the histogram of CD11b⁺ bone marrow cell counts analyzed by flow cytometry.
FIG. 8 is a correlation diagram showing the progression of the disease course of CRLM complicated by NAFLD and the massive expansion of CD11b⁺Gr1⁺ MDSCs.
FIG. 9 shows the expression of myeloid cells in the livers of each group of mice.
FIG. 10 shows the results that reprixin treatment reduces liver metastasis burden in mice complicated by NAFLD.
FIG. 11 shows the expression of CD45⁺/CD11b⁺/Gr1⁺ MDSCs in the livers of each group of mice on day 12.
FIG. 12 shows that the treatment with reparixin in combination with the anti-PD-1 antibody can reduce the liver metastasis burden in mice complicated by NAFLD.
FIG. 13 shows that the two-drug treatment can alleviate the condition of mice with liver metastasis complicated by NAFLD.
FIG. 14 shows the relative quantification of CD8⁺ in the livers of each treatment group of mice.
FIG. 15 shows the treatment effects of reparixin on NAFLD on days 12 and 15.
FIG. 16 shows the treatment effect of reparixin on NAFLD on day 21. A: representative gross views of livers of mice in the NAFLD treatment group and untreated group, and oil red O staining and immunohistochemical staining on liver sections of the two groups of mice, with the scale bar being 100 µm; B-C: comparison of mean immunohistochemical staining densities of IL6 (B) and CXCR2 (C) in livers of NAFLD mice treated with reparixin for 21 days (mean ± SD, Student's t test).
FIG. 17 shows the effects of the treatment with reparixin in combination with PD-1 on mice with liver metastasis from lung cancer complicated by NAFLD or uncomplicated by NAFLD after 12 days, including gross anatomical views of livers of each group of mice (arrows represent tumors, left panel) and statistical results of liver/body weight ratio (right panel).

### DETAILED DESCRIPTION

The technical solutions of the present disclosure are further described in detail below with reference to specific examples. It will be appreciated that the following examples are merely exemplary illustrations and explanations of the present disclosure and should not be construed as limiting the claimed scope of the present disclosure. All techniques implemented on the basis of the content described above of the present disclosure fall within the claimed scope of the present disclosure. Unless otherwise stated, the raw materials and reagents used in the following examples are all commercially available products or can be prepared by using known methods.

### Example 1. Establishment of NAFLD, CRLM, and NAFLD + CRLM Animal Models

### 1. Establishment of mouse NAFLD + liver metastasis by intra-splenic injection dual model

1.1 NAFLD modeling (J Hepatol. 2018 Apr; 67:1303): The "Western diet" (WD) was simulated with 60% high-fat feed, and the NAFLD model could be established within 12 weeks; after NAFLD was formed, CRLM on the basis of NAFLD was established by intra-splenic injection of homologous mouse CRC colorectal cancer cells for the subsequent study.

1.2 CRLM modeling (Hepatology 2013; 57:829-839): On the basis of 1.1, the mice were anesthetized, and then an abdominal incision was made; 5 × 10⁵ MC38 or other C57 background CRC cells were intrasplenically injected; 30 s after injection, the spleen was excised, and the abdominal incision was closed by suturing. The mice were sacrificed on days 10-14 as required for the experiment, and samples were collected.

### 2. Establishment of CRLM animal model

2.1 Ectopic implantation model: Hepatic portal vein injection was used for modeling. After anesthesia with isoflurane was successful, the mice were fixed on an operating table. The skin was disinfected conventionally. A midline incision was made in the abdomen, with a length of about 1 cm, to expose the hepatic portal vein. A 0.2 mL syringe (31G needle) was inserted into the hepatic portal vein at 30° to the horizontal. 100 µL of MC38 cells or CT26 cells was injected. After the needle was pulled out, an alcohol cotton ball was pressed against the puncture site for about 10 min. After no bleeding was observed, the muscle and skin were sutured intermittently in sequence, and the abdomen was closed.

2.2 Orthotopic implantation: After anesthesia with isoflurane was successful, the mice were fixed on an operating table. The skin was disinfected conventionally. A transverse incision was made in the lower abdomen, and a CRC tumor mass with a size of about 1 mm³ was sutured to the head end of the cecum using a purse-string suture with 5-0 silk thread.

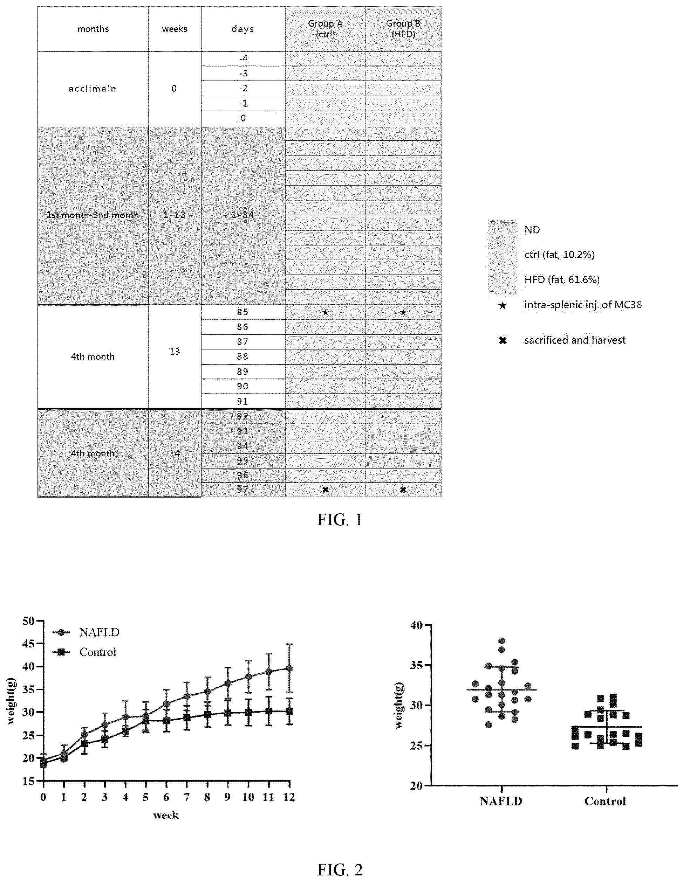

FIG. 1 is a flowchart of modeling of the mouse NAFLD + CRC liver metastasis dual model.

FIG. 2 shows the body weight of the NAFLD modeling mice; left: the dynamic change of the modeling body weight of the mice in the NAFLD group and the control group; right: the comparison of the body weight of the mice in the NAFLD group and the control group after modeling.

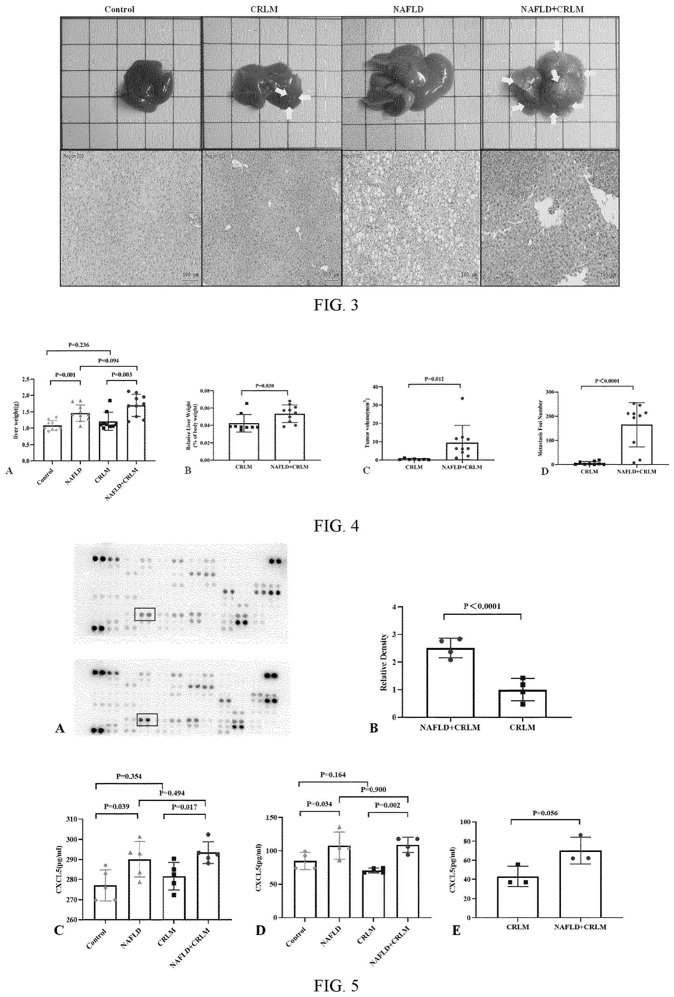

FIG. 3 shows the gross anatomical views of livers (upper panel) and HE staining (lower panel) of each group of mouse models; yellow arrows represent liver metastases, and blue arrows represent fatty liver lesion sites; NAFLD tumor-bearing group (NAFLD + CRLM), 60% high-fat feed + MC38 intra-splenic injection; NAFLD non-tumor-bearing group (NAFLD), 60% high-fat feed + PBS intra-splenic injection; control tumor-bearing group (CRLM), 10% fat feed + MC38 intra-splenic injection; control non-tumor-bearing group (Control), 10% fat feed + PBS intra-splenic injection.

FIG. 4 shows a comparative analysis of liver metastases of mice across the groups; A. comparison of absolute liver weight of the groups (mean ± SD, one-way ANOVA); B. comparison of liver-to-body weight ratios of tumor-bearing groups; C. comparison of maximum tumor volume of tumor-bearing groups; D. comparison of the number of liver metastases of tumor-bearing groups (mean ± SD, Student's t-test).

As can be seen from FIGs. 1-4, mouse models of NAFLD, CRLM, and NAFLD + CRLM were successfully established.

### Example 2. Study on Localization of Chemokines in CRLM Complicated by NAFLD

In metastatic foci and peritumoral liver tissues from CRLM specimens of clinical cases complicated by NAFLD or mouse models, the expression of candidate chemokines was detected. Detection specimens: paraffin sections or fresh snap-frozen tissue sections of CRLM lesions from clinical cases or mouse models.

Detection method: immunohistochemistry (IHC) (experimental technique as described in the applicant's previously published article: Hepatology. 2013;57:829), ELISA (Abcam), Mouse XL Cytokine Array Kit (R&D, ARY028).

FIG. 5 shows the screening and identification of chemokines: (A) Mouse XL Cytokine Array assay for various cytokines, chemokines, growth factors, and other soluble proteins in serum. The data were obtained 5 min after exposure by Bio-Rad (upper panel: CRLM group, lower panel: CRLM complicated by NAFLD group); (B) relative levels of CXCL5 in peripheral blood of each tumor-bearing C57BL/6 mouse model, n = 4; (C) quantification of the CXCL5 concentration in peripheral blood of each group of mice, n = 5; (D) quantification of the CXCL5 concentration in liver homogenate of each group of mice, n = 4; (E) quantitative analysis of the CXCL5 concentration in tumor tissues of each tumor-bearing C57BL/6 mouse model, n = 3.

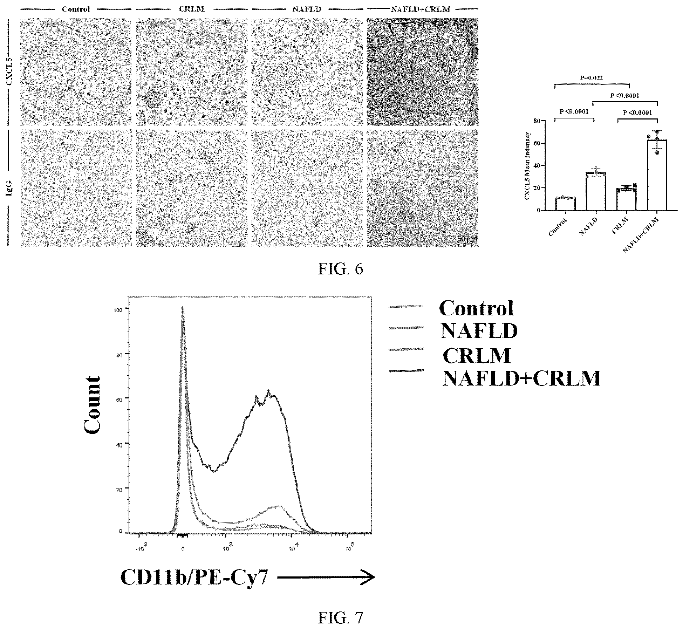

FIG. 6 shows the expression of CXCL5 in the livers of each group of mice. Left panel: representative IHC images of CXCL5 in liver tissues of each group of mice. Bar, 50 µm; right panel: mean expression intensity of CXCL5 in liver tissues analyzed by the TG tissue cell imaging system. Mean ± SD, one-way ANOVA.

As can be seen from FIGs. 5-6, the expression level of CXCL5 in NAFLD and NAFLD + CRLM animal models was higher than that in the control group and the CRLM animal model.

### Example 3. Detection and Identification of Infiltration of Corresponding Chemokine Receptor-Positive Cells in CRLM Complicated by NAFLD

### 3.1 Detection of expression of corresponding chemokine receptors in liver tissues of each group of mice

Detection specimens: paraffin sections or fresh snap-frozen tissue sections of CRLM lesions from clinical cases or mouse models.

### Detection method: immunohistochemistry (IHC).

3.2 Isolation and identification of types of corresponding chemokine receptor-positive cells.

Experimental specimens: fresh specimens of CRLM lesions from clinical cases or mouse models

Experimental methods: Flow cytometry sorting (FACS sorting) and single-cell sequencing: Receptor-positive cells were sorted by the flow cytometer, and their cell types were identified by their molecular phenotypes. It was speculated that the chemotactic cells were most likely myeloid cells; therefore, markers of myeloid cells, such as CD11b, were detected first. Single-cell suspensions were loaded into 10× Chromium, and single cells were captured according to the manufacturer's instructions for the 10× Genomics Chromium single-cell 3' kit (V3). cDNA amplification and library construction steps were performed according to standard protocols. The libraries were sequenced on the Illumina NovaSeq 6000 sequencing system (150 bp) of LC-Bio Technology Co., Ltd. (Hangzhou, China), and re-verified by the method described above.

### 3.3 Verification of results described above in clinical specimens: immunohistochemistry (IHC)

FIG. 7 shows the histogram of CD11b+ bone marrow cell counts analyzed by flow cytometry.

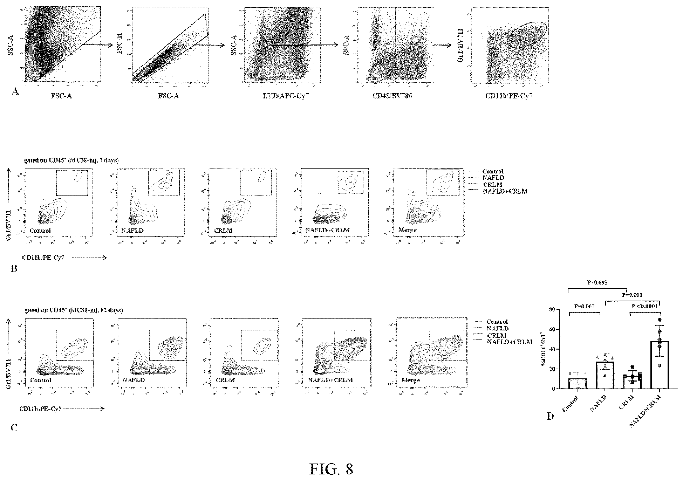

FIG. 8 is a correlation diagram showing the progression of the disease course of CRLM complicated by NAFLD and the massive expansion of CD11b⁺Gr1⁺ MDSCs. (A) Gating strategy in flow cytometry. The mice were sacrificed under isoflurane anesthesia, and the livers were soaked in alcohol for 30 min and collected on days 7 (B) and 12 (C) after MC38 inoculation, respectively, for flow cytometry analysis (D).

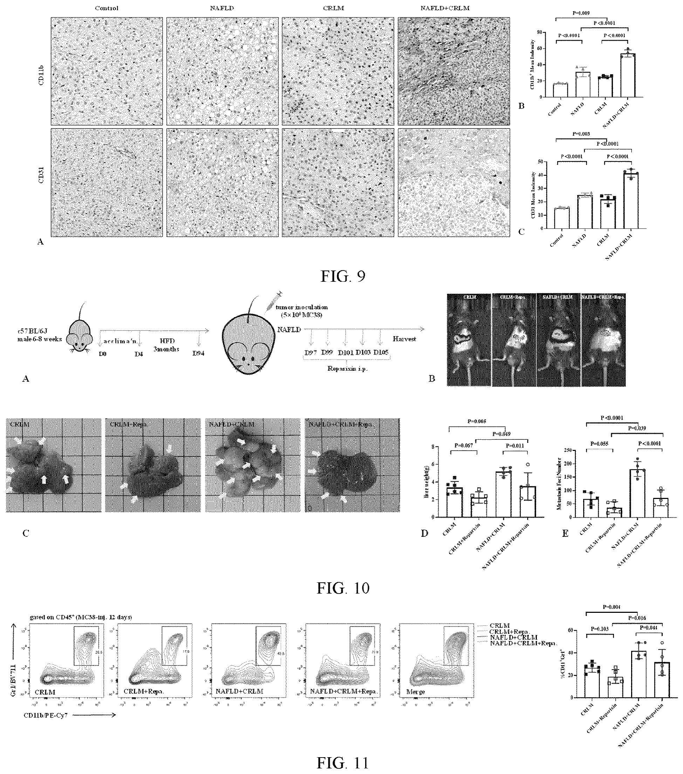

FIG. 9 shows the expression of hepatic myeloid cells of each group of mice, (A) representative IHC images of CD11b⁺ and CD31⁺ in liver tissues of each group of mice. Bar, 50 µm; (B-C) mean expression intensity of CD11b ⁺ and CD31⁺ in liver tissues analyzed by the TG tissue cell imaging system. Mean ± SD, one-way ANOVA.

As can be seen from FIGs. 7-9, compared with the control group, the expression of hepatic myeloid cells was enhanced in the CRLM animal model complicated by NAFLD, NAFLD animal model, and CRLM animal model. Among them, the level of MDSCs in the liver was the highest in the CRLM animal model complicated by NAFLD, followed by that in the NAFLD animal model. This suggests that the infiltration of MDSCs recruited into the liver was increased in the context of NAFLD, which further aggravates the liver metastasis burden.

### Example 4. In Vivo Study of Effect of Reparixin on CRC Liver Metastasis Using Mouse Liver Metastasis Model by Intra-Splenic Injection

Experimental methods: Luciferase-MC38 cells were intrasplenically injected into C57BL/6 mice complicated by NAFLD or uncomplicated by NAFLD. Subsequently, reparixin (MCE, 30 mg/kg) or PBS was intraperitoneally injected every other day. On day 6 after tumor inoculation, the extent of liver metastasis was observed by imaging using an *In vivo* Imaging System (IVIS, PerkinElmer, Hopkinton, MA, USA). On day 12 after tumor inoculation, the mice were sacrificed under isoflurane anesthesia and soaked in alcohol for 30 min. Livers from each group of mice were prepared into single-cell suspensions, which were subjected to blocking, staining, incubation, and subsequent detection on the machine.

Experimental grouping: The experimental groups were injected with reparixin, and the control group was injected with PBS.

FIG. 10 shows the results that reprixin treatment reduces liver metastasis burden in mice complicated by NAFLD. (A) Experimental treatment protocol: After Luciferase-MC38 cells were inoculated, PBS or reparixin was injected on days 3, 5, 7, 9, and 11, respectively, and the mice were sacrificed on day 12. (B) *In vivo* bioluminescence imaging of each group (CRLM group, CRLM + reparixin group, NAFLD + CRLM group, NAFLD + CRLM + reparixin group) on day 6 after tumor inoculation. (C) Representative gross anatomical views of livers of each group of mice (samples collected on day 12 after treatment); yellow arrows represent tumors. (D) Liver weight of each group of mice; n = 5-6.

### (E) Liver/body weight ratio of each group of mice; n = 5-6.

FIG. 11 shows the expression of CD45⁺/CD11b⁺/Gr-1⁺ MDSCs in the livers of each group of mice on day 12, left panel: representative histogram; right panel: flow cytometry analysis of the percentage (%) of MDSCs (CD45+C11b+Gr-1+) to CD45+ cells.

As can be seen from FIGs. 10-11, after the reparixin treatment, the liver weight and liver/body weight ratio in the CRLM and NAFLD + CRLM animal models decreased significantly. This indicates that reparixin can reduce the liver metastasis burden, especially the liver metastasis burden in mice complicated by NAFLD.

### Example 5. Effect of Treatment with Reparixin in Combination with Anti-PD-1 on CRC Liver Metastasis in Mice Complicated by NAFLD

Experimental methods: Luciferase-MC38 cells were intrasplenically injected into C57BL/6 mice complicated by NAFLD. On day 3 after inoculation, the mice were randomized into treatment groups to observe the overall survival (OS) rate, and the study was terminated when the mice had poor activity or lost more than 20% from their initial postsurgical body weight according to the ARENA/OLAW IACUC guidelines. Tumor growth was detected by bioluminescence imaging IVIS 12 days after the inoculation of Luciferase-MC38 cells; the growth of liver metastases in the two-drug groups was detected by IVIS 58 days after the inoculation of Luciferase-MC38 cells. Liver tissues from each group were subjected to HE staining and multiplex immunofluorescence staining. Experimental grouping: reparixin treatment group: intraperitoneal injection once every 2 days at a dose of 30 mg/kg; PD-1 treatment group: anti-mouse PD-1 monoclonal antibody (mAb, 200 µg/mouse/day; #BE0146, BioXcell, USA) administered by intraperitoneal injection starting from day 3 (once every 2 days); two-drug treatment group: starting from day 3, anti-pd-1 mAb (200 µg/mouse/day) administered intraperitoneally once every 2 days, with reparixin (30 mg/kg) given on the same day; control group: daily injection of an equivalent amount of an isotype IgG antibody.

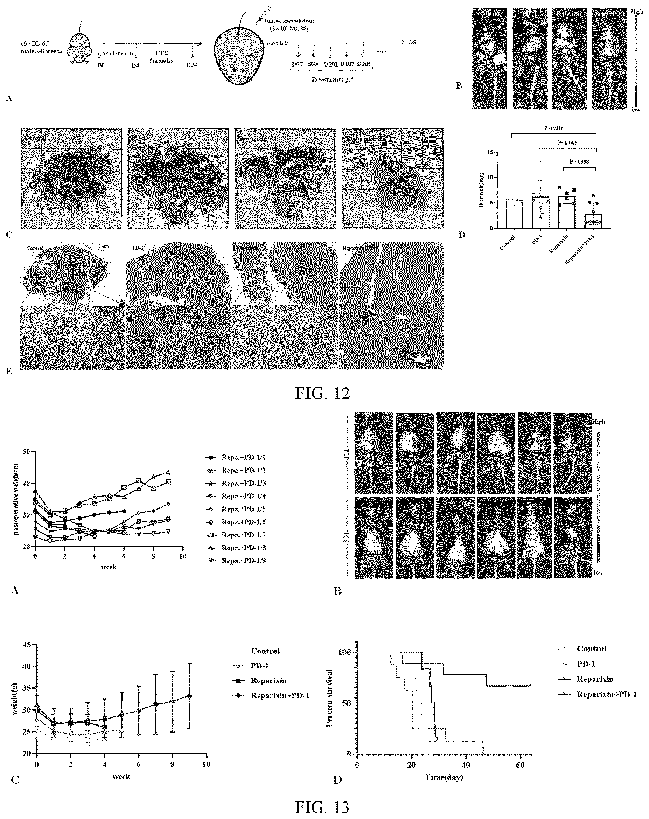

Experimental results: The formation of liver metastases and the corresponding immune cell infiltration in the liver microenvironment were compared. FIG. 12 shows that the treatment with reparixin in combination with the PD-1 antibody can reduce the liver metastasis burden in mice complicated by NAFLD.

(A) Different treatment regimens. * Intraperitoneal injection treatment. After the inoculation of Luciferase-MC38 cells, the isotype IgG, PD-1 monoclonal antibody, reparixin, and combination treatment (reparixin + PD-1) were separately injected every other day starting from day 3; (B) *in vivo* bioluminescence imaging of each treatment group (NAFLD + CRLM + isotype-IgG, NAFLD + CRLM + PD-1, NAFLD + CRLM + reparixin, NAFLD + CRLM + PD-1 + reparixin) on day 12 after the inoculation of Luciferase-MC38. n = 6-9; (C) gross anatomical views of livers in each treatment group. n = 6-9; (D) relative liver weight of each group of mice. Samples were collected when endpoint events occurred, n = 6-9; (E) representative images of HE staining of mouse liver sections in each treatment group, with boxes indicating lymphocytes. Bar, 1 mm: upper panel; 100 µm: lower panel.

FIG. 13 shows that the two-drug treatment can alleviate the condition of mice with liver metastasis complicated by NAFLD.

(A)The trend of change in the body weight of mice in the combination treatment groups. n = 9; (B) *in vivo* bioluminescence imaging on day 58 of the combination treatment groups. n = 6; (C) the trend of change in the body weight of mice under different treatment regimens. n = 6-9; (D) survival curves of mice. n = 6-9, Log-rank (Mantel-Cox) test was performed with NAFLD + CRLM + isotype IgG group.

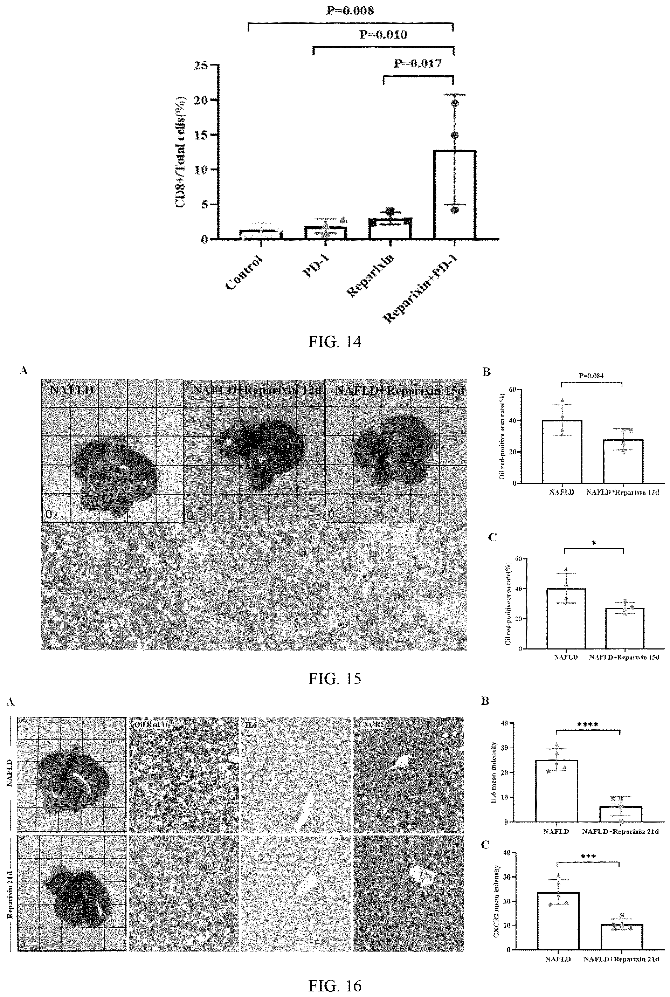

FIG. 14 shows the relative quantification of CD8⁺ in the livers of each treatment group of mice.

As can be seen from FIGs. 12-14, the treatment with reparixin in combination with the PD-1 antibody can reduce the liver metastasis burden in mice complicated by NAFLD, with a significantly better effect than reparixin or the PD-1 antibody alone. Considering the results of clinical trials evaluating anti-PD-1 treatment for CRC, it has been demonstrated that only about 15% of CRC patients with deficient mismatch repair (dMMR) benefit from the treatment. To improve the treatment effect, CD8⁺ T cells were increased after the treatment with reparixin in combination with PD-1 on a preliminary basis, which indicates that the success in inhibiting metastasis in cancer treatment depends on massive T lymphocyte infiltration.

### Example 6. In Vivo Study of Effect of Reparixin on NAFLD

Experimental methods: The "Western diet" (WD) was simulated with 60% high-fat feed, and the NAFLD model could be established within 12 weeks. After the establishment of the animal model, the mice were intraperitoneally injected once every 2 days at a dose of 30 mg/kg. Liver tissues of the mice were collected on days 12, 15, and 21 of administration, and oil red staining was performed on the liver tissues of each group.

Experimental grouping: The experimental groups were injected with reparixin, and the control group was injected with PBS.

FIG. 15 shows different treatment effects on NAFLD after 12 d or 15 d of reparixin administration; FIG. 16 shows different treatment effects on NAFLD after 21 d of reparixin administration.

FIG. 15 shows different treatment effects of reparixin on NAFLD on day 12 or day 15. (A) After NAFLD was established, raparixin was intraperitoneally injected once every two days; macroscopic views of representative liver tissues and oil red staining of liver sections in each group are shown, and the scale represents 50 µm. (B) Oil red staining positive area rate of NAFLD and 12-day treatment groups, n = 4. (C) Oil red staining positive area rate of NAFLD and 15-day treatment groups, n = 4. Statistical test: mean + SD, Student's t-test, * p < 0.05.

FIG. 16 shows the treatment effect of reparixin on NAFLD on day 21. A: representative gross views of livers of mice in the NAFLD treatment group and untreated group, and oil red O staining and immunohistochemical staining on liver sections of the two groups of mice, with the scale bar being 100 µm; B-C: comparison of mean immunohistochemical staining densities of IL6 (B) and CXCR2 (C) in livers of NAFLD mice treated with reparixin for 21 days (mean ± SD, Student's t test).

It can be seen from the analysis of FIGs. 15-16 that reparixin can ameliorate hepatic steatosis and reduce inflammation, and can be used for the treatment of NAFLD. With the progression of the reparixin treatment, hepatocellular steatosis in NAFLD was significantly ameliorated, and lipid vacuoles were reduced, suggesting that reparixin can reverse fatty liver to a certain extent.

### Example 7. Treatment of Liver Metastasis from Lung Cancer LCLM by Reparixin in Combination with PD-1

Experimental grouping: Mice with liver metastasis from lung cancer (LCLM) complicated by NAFLD or uncomplicated by NAFLD were divided into a two-drug treatment group (reparixin + PD-1 monoclonal antibody) and an IgG control group.

Experimental methods: On the basis of mice complicated by NAFLD or uncomplicated by NAFLD, an abdominal incision was made after anesthesia, and 5 × 10⁵ LLC mouse lung cancer cells were intrasplenically injected; 30 s after injection, the spleen was excised, and the abdominal incision was closed by suturing. Starting from day 3 after tumor inoculation, reparixin (MCE, 30 mg/kg) and anti-mouse PD-1 monoclonal antibody (mAb, 200 µg/mouse/day; #BE0146, BioXcell, USA) were administered by intraperitoneal injection once every two days; the control group received an equivalent amount of an isotype IgG antibody daily. The mice were sacrificed on day 14 as required for the experiment, and samples were collected.

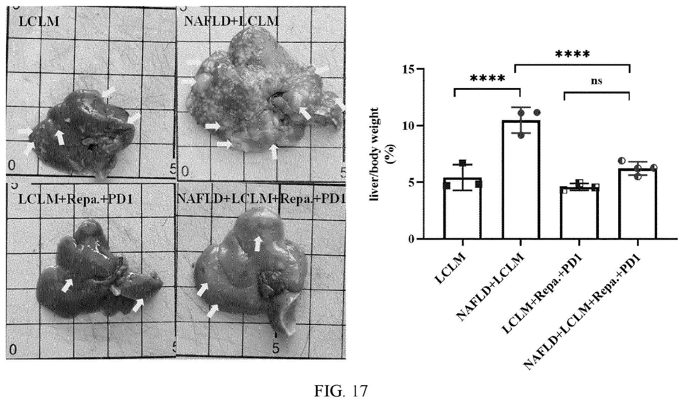

Experimental results: As shown in FIG. 17, after 12 days of treatment with reparixin in combination with PD-1, the tumor burden was alleviated to a certain extent in mice with liver metastasis from lung cancer complicated by NAFLD or uncomplicated by NAFLD; among them, the two-drug combination treatment can significantly alleviate the tumor burden of LCLM in mice complicated by NAFLD.

### REFERENCES

1. Zhou F, Zhou J, Wang W, Zhang XJ, Ji YX, Zhang P, She ZG, Zhu L, Cai J, Li H: Unexpected Rapid Increase in the Burden of NAFLD in China From 2008 to 2018: A Systematic Review and Meta-Analysis. Hepatology 2019, 70(4):1119-1133.
2. Fan JG, Kim SU, Wong VW: New trends on obesity and NAFLD in Asia. J Hepatol 2017, 67(4):862-873.
3. Younossi ZM, Koenig AB, Abdelatif D, Fazel Y, Henry L, Wymer M: Global epidemiology of nonalcoholic fatty liver disease-Meta-analytic assessment of prevalence, incidence, and outcomes. Hepatology 2016, 64(1):73-84.
4. Estes C, Anstee QM, Arias-Loste MT, Bantel H, Bellentani S, Caballeria J, Colombo M, Craxi A, Crespo J, Day CP, Eguchi Y, Geier A, Kondili LA, Kroy DC, Lazarus JV, Loomba R, Manns MP, Marchesini G, Nakajima A, Negro F, Petta S, Ratziu V, Romero-Gomez M, Sanyal A, Schattenberg JM, Tacke F, Tanaka J, Trautwein C, Wei L, Zeuzem S, Razavi H: Modeling NAFLD disease burden in China, France, Germany, Italy, Japan, Spain, United Kingdom, and United States for the period 2016-2030. J Hepatol 2018, 69(4):896-904.
5. Huo T, Cao J, Tian Y, Shi X, Wu L, Zhang M, Wong LL, Zhao L: Effect of Concomitant Positive Hepatitis B Surface Antigen on the Risk of Liver Metastasis: A Retrospective Clinical Study of 4033 Consecutive Cases of Newly Diagnosed Colorectal Cancer. Clin Infect Dis 2018, 66(12):1948-1952.
6. Yang Y, Song L, Cao J, Liu J, Wang D, Wong LL, Zhao L: Active Chronic Hepatitis B increases the risk of Colorectal Liver Metastasis - A retrospective cross-sectional study. J Cancer 2021, 12(5):1398-1405.
7. Siriwardena AK, Mason JM, Mullamitha S, Hancock HC, Jegatheeswaran S: Management of colorectal cancer presenting with synchronous liver metastases. Nat Rev Clin Oncol 2014, 11(8):446-459.
8. Yin Z, Liu C, Chen Y, Bai Y, Shang C, Yin R, Yin D, Wang J: Timing of hepatectomy in resectable synchronous colorectal liver metastases (SCRLM): Simultaneous or delayed? Hepatology 2013, 57(6):2346-2357.
9. Ramos E, Torras J, Llado L, Rafecas A, Serrano T, Lopez-Gordo S, Busquets J, Fabregat J: The influence of steatosis on the short- and long-term results of resection of liver metastases from colorectal carcinoma. HPB (Oxford) 2016, 18(4):389-396.
10. Hamady ZZ, Rees M, Welsh FK, Toogood GJ, Prasad KR, John TK, Lodge JP: Fatty liver disease as a predictor of local recurrence following resection of colorectal liver metastases. Br J Surg 2013, 100(6):820-826.
11. Lv Y, Patel N, Zhang HJ: The progress of non-alcoholic fatty liver disease as the risk of liver metastasis in colorectal cancer. Expert Rev Gastroenterol Hepatol 2019, 13(12):1169-1180.
12. Koopman M, Kortman GA, Mekenkamp L, Ligtenberg MJ, Hoogerbrugge N, Antonini NF, Punt CJ, van Krieken JH: Deficient mismatch repair system in patients with sporadic advanced colorectal cancer. Br J Cancer 2009, 100(2):266-273.
13. Topalian SL, Hodi FS, Brahmer JR, Gettinger SN, Smith DC, McDermott DF, Powderly JD, Carvajal RD, Sosman JA, Atkins MB, Leming PD, Spigel DR, Antonia SJ, Horn L, Drake CG, Pardoll DM, Chen L, Sharfman WH, Anders RA, Taube JM, McMiller TL, Xu H, Korman AJ, Jure-Kunkel M, Agrawal S, McDonald D, Kollia GD, Gupta A, Wigginton JM, Sznol M: Safety, activity, and immune correlates of anti-PD-1 antibody in cancer. N Engl J Med 2012, 366(26):2443-2454.
14. Brahmer JR, Tykodi SS, Chow LQ, Hwu WJ, Topalian SL, Hwu P, Drake CG, Camacho LH, Kauh J, Odunsi K, Pitot HC, Hamid O, Bhatia S, Martins R, Eaton K, Chen S, Salay TM, Alaparthy S, Grosso JF, Korman AJ, Parker SM, Agrawal S, Goldberg SM, Pardoll DM, Gupta A, Wigginton JM: Safety and activity of anti-PD-L1 antibody in patients with advanced cancer. N Engl J Med 2012, 366(26):2455-2465.
15. Wang Y, Lin HC, Huang MY, Shao Q, Wang ZQ, Wang FH, Yuan YF, Li BK, Wang DS, Ding PR, Chen G, Wu XJ, Lu ZH, Li LR, Pan ZZ, Sun P, Yan SM, Wan DS, Xu RH, Li YH: The Immunoscore system predicts prognosis after liver metastasectomy in colorectal cancer liver metastases. Cancer Immunol Immunother 2018, 67(3):435-444.
16. Katz SC, Bamboat ZM, Maker AV, Shia J, Pillarisetty VG, Yopp AC, Hedvat CV, Gonen M, Jarnagin WR, Fong Y, D'Angelica MI, DeMatteo RP: Regulatory T cell infiltration predicts outcome following resection of colorectal cancer liver metastases. Ann Surg Oncol 2013, 20(3):946-955.
17. Donadon M, Torzilli G, Cortese N, Soldani C, Di Tommaso L, Franceschini B, Carriero R, Barbagallo M, Rigamonti A, Anselmo A, Colombo FS, Maggi G, Lleo A, Cibella J, Peano C, Kunderfranco P, Roncalli M, Mantovani A, Marchesi F: Macrophage morphology correlates with single-cell diversity and prognosis in colorectal liver metastasis. J Exp Med 2020, 217(11).
18. Cavnar MJ, Turcotte S, Katz SC, Kuk D, Gonen M, Shia J, Allen PJ, Balachandran VP, D'Angelica MI, Kingham TP, Jarnagin WR, DeMatteo RP: Tumor-Associated Macrophage Infiltration in Colorectal Cancer Liver Metastases is Associated With Better Outcome. Ann Surg Oncol 2017, 24(7):1835-1842.
19. Xin B, Yang M, Wu P, Du L, Deng X, Hui E, Feng GS: Enhancing the therapeutic efficacy of programmed death ligand 1 antibody for metastasized liver cancer by overcoming hepatic immunotolerance in mice. Hepatology 2022, 76(3):630-645.
20. Kim HY, Choi JH, Kang YJ, Park SY, Choi HC, Kim HS: Reparixin, an inhibitor of CXCR1 and CXCR2 receptor activation, attenuates blood pressure and hypertension-related mediators expression in spontaneously hypertensive rats. Biol Pharm Bull 2011, 34(1):120-127.
21. Sousa LF, Coelho FM, Rodrigues DH, Campos AC, Barcelos Lda S, Teixeira MM, Rachid MA, Teixeira AL: Blockade of CXCR1/2 chemokine receptors protects against brain damage in ischemic stroke in mice. Clinics (Sao Paulo) 2013, 68(3):391-394.
22. Lin C, He H, Liu H, Li R, Chen Y, Qi Y, Jiang Q, Chen L, Zhang P, Zhang H, Li H, Zhang W, Sun Y, Xu J: Tumour-associated macrophages-derived CXCL8 determines immune evasion through autonomous PD-L1 expression in gastric cancer. Gut 2019, 68(10):1764-1773.
23. Li X, Yao W, Yuan Y, Chen P, Li B, Li J, Chu R, Song H, Xie D, Jiang X, Wang H: Targeting of tumour-infiltrating macrophages via CCL2/CCR2 signalling as a therapeutic strategy against hepatocellular carcinoma. Gut 2017, 66(1):157-167.
24. Loberg RD, Ying C, Craig M, Yan L, Snyder LA, Pienta KJ: CCL2 as an important mediator of prostate cancer growth in vivo through the regulation of macrophage infiltration. Neoplasia 2007, 9(7):556-562.

The embodiments of the present disclosure have been described above. However, the embodiments described above are not intended to limit the present disclosure. Any modification, equivalent replacement, improvement, and the like made without departing from the spirit and principle of the present disclosure shall fall within the protection scope of the present disclosure.

## Claims

1. Use of reparixin or a pharmaceutically acceptable salt thereof for the manufacturing of a medicament for the treatment and/or prevention of colorectal cancer liver metastasis CRLM and liver metastasis from lung cancer.

2. The use according to claim 1, wherein the colorectal cancer liver metastasis is colorectal cancer liver metastasis complicated by NAFLD;
preferably, the liver metastasis from lung cancer is liver metastasis from lung cancer complicated by NAFLD.

3. Use of reparixin or a pharmaceutically acceptable salt thereof for the manufacturing of a medicament for the treatment and/or prevention of non-alcoholic fatty liver disease NAFLD.

4. The use according to claim 3, wherein
the non-alcoholic fatty liver disease comprises primary non-alcoholic fatty liver disease and secondary non-alcoholic fatty liver disease;
and/or the non-alcoholic fatty liver disease comprises simple fatty liver, non-alcoholic steatohepatitis, and non-alcoholic steatohepatitis-related cirrhosis.

5. A pharmaceutical combination composition, comprising an anti-PD-1 antibody or an antigen-binding fragment thereof and reparixin or a pharmaceutically acceptable salt thereof.

6. The use according to claim 5, wherein the anti-PD-1 antibody or the antigen-binding fragment thereof has a synergistic pharmacodynamic effect with reparixin or the pharmaceutically acceptable salt thereof.

7. Use of the pharmaceutical combination composition according to claim 5 or 6 for the manufacturing of a medicament for the treatment and/or prevention of a cancer.

8. The use according to claim 7, wherein the cancer is liver cancer, colorectal cancer, colon cancer, or rectal cancer; preferably, the cancer is metastatic liver cancer or secondary liver cancer; preferably, the metastatic liver cancer comprises colorectal cancer liver metastasis CRLM, breast cancer liver metastasis, ovarian cancer liver metastasis, neuroendocrine cancer liver metastasis, and liver metastasis from lung cancer, such as colorectal cancer liver metastasis complicated by NAFLD and liver metastasis from lung cancer complicated by NAFLD.
